# EUROPEAN PATENT APPLICATION

(11) **EP 3 657 414 A1**
(43) Date of publication of application: **27.05.2020**
(21) Application number: 18834464.2
(22) Date of filing: 17.07.2018
(51) Int. Cl.: G06Q 10/08, G06Q 30/02, G07F 11/00, A47B 67/02

(54) **AVAILABILITY OF MEDICINAL PRODUCTS**

(30) Priority: 17.07.2017 EP 17181593
(71) Applicant: Bayer S.A., 04779-900 Sao Paulo SP (BR)
(72) Inventor: DUY, Tam Nguyen, Ho Chi Minh (VN); GANT-FLEENER, Emerson, Granger, IN 46530 (US); FU, Felipe Bechert, 05441-000 São Paulo (BR); MOLINA SOTO, Karen, Ciudad De Panamá (PA); SANTOS, Leonardo Junio de Oliveira, 01226-000 São Paulo (BR)
(74) Representative: BIP Patents
(86) International application number: PCT/BR2018/050246
(87) International publication number: WO 2019/014742

(57) **Abstract**

The present invention relates to a system, a process, a computer program product and a storage unit that allow the availability of medicinal products in a point of sale to be checked. People who wish or need to buy a medicinal product for a chronic or acute disease normally need this medicinal product immediately.

## Description

The present invention refers to a system, a process, a product for a computer program and a storage unit by means whereof the availability of medicinal products at a point of sale may be examined.

Persons who would like/need to purchase a medicinal product by virtue of a chronic or acute disease normally require this medicinal product immediately.

A person requiring a medicinal product may go to a corresponding point of sale (for example, a chemist's shop) to obtain or to ask for the medicinal product in that premises. Frequently, it occurs that the shop (a point of sale) does not have the required medicinal product available in stock. When a medicinal product is not available in stock, currently it may be acquired with great rapidity, in general less than 24 hours. However for those requiring the medicinal product, and each hour of waiting for a medicinal product ordered may make a great difference, they have the choice of ordering the required medicinal product or obtaining it in another shop; on selecting the second option they will not know, in general, whether the other shop will have the medicinal product desired available in stock.

Many shops currently offer upon their Internet pages the possibility of consulting the availability of a product via the Internet. This procedure would offer the person desirous of purchasing a medicinal product the advantage that they may firstly search on the Internet as to whether the medicinal product sought is available in a shop; it also saves going fruitlessly to a shop. However, often, they will require to consult many pages of diverse shops on the Internet to identify the shop having the desired medicinal product available in stock. Habitually, the Internet pages of diverse shops do not dispose of a uniform configuration and the person seeking the availability of a medicinal product must reorient themselves upon each consultation of an Internet page.

Another problem consists in the fact that the manufacturer and/or distributor of the products also does not always know where the products thereof are precisely situated. Very often, the medicinal products are distributed by a network of intermediary traders prior to reaching the retail shop whereat they may be acquired by a client. However, the manufacturer and/or distributor of medicinal products does not know how many of the products thereof left the factory thereof, have reached the retail shop, and how they are being distributed in the premises, that is to say which retail shop has which quantities available in stock. Nevertheless, this information may be of value to optimize the distribution and the logistics of the medicinal products.

These problems illustrated are solved through the objects of the independent claims. The preferred embodiments may be found in the subordinate claims, in the description below, and in the figures.

A first object of the invention is a system encompassing
- a multiplicity of storage units of units of medicinal products,
   wherein the storage units dispose of resources to register the quantity of the units of medicinal products available in the respective storage unit,
   wherein the storage units are connected in a communicative manner to a first computerized system and are configured to transmit the respective quantities of the units of medicinal products available in the storage units to the first computerized system,
- the first computerized system,
   being connected in a communicative manner to the storage units and being configured to receive from the storage units the quantities of the units of medicinal products available in the storage units and to register them in a data base,
- the data base,
   wherein are registered the premises whereat are situated the storage units and the respective quantities of the units of medicinal products available received for those storage units,
- at least a second computerized system,
   connected in a communicative manner to the first computerized system and/or to the data base by means of a network and being configured such that a user, subsequent to the input and/or selection of a premises or subsequent to the input of the premises wherein the user is precisely situated, or subsequent to the inputting of the information regarding a medicinal product or subsequent to the selection of a medicinal product, may receive the readout of the premises of the storage units situated in the defined vicinity of the premises and the quantity of the units of medicinal products available in the storage units.

Another object of the invention is a process comprising the stages:
- Inputting and/or selection of a medicinal product by a user,
- Inputting and/or selection of a premises by a user and/or inputting of the premises wherein the user is situated,
- Readout of the premises in a defined vicinity of the premises, it being that there are situated in the premises the storage units disposing of the medicinal product in relation to the user, or readout of the quantity of units of medicinal products in the respective storage units in relation to the user.

A yet another object of the invention is a process encompassing the stages:
- Availability of storage units containing units of medicinal products in diverse premises,
- Automated identification of the quantities of units of medicinal products situated in the storage units,
- Transmission of the quantities to a first computerized system,
- Registration of the quantities together with the premises in a data base,
- Updating of the data base in relation to the defined moments and/or in the case of registration of defined occurrences,
- Rendering available an access to the first computerized system and/or to the data base by a user by means of a second computerized system, wherein the access is configured such that, subsequent to the inputting and/or selection and/or inputting of a premises, the user may have the readout of the premises having storage units situated in a defined vicinity of the premises, and wherein the user may additionally read the quantities of the units of medicinal products available in that premises.

A further objective of the invention is a product for a computer program encompassing a data registration device wherein is stored a computer program capable of being loaded into the memory of a computerized system and which, by means of that memory, the computerized system permits executing the following stages:
- Determination of a premises,
- Determination of the information referring to a medicinal product,
- Determination of the premises in a defined vicinity of the premises whereat are situated the storage units of the medicinal product,
- Determination of the quantities of the units of medicinal products situated in the storage units,
- Readout of the premises and of the quantities of units of medicinal products available in the premises in relation to a user.

A yet further object of the invention is a storage unit of the units of medicinal products, wherein the storage unit disposes of resources to register the quantity of the units of medicinal products available in the storage unit,
wherein the storage unit is connected in a communicative manner to a first computerized system and is configured such as to be enabled to transmit to the first computerized system the quantity of the unit of medicinal product available in the storage unit.

The invention is clarified below in detail without distinction being made between the objects of the invention (process, system, product for computer program, storage unit). On the contrary, the clarifications below shall be applied to all the objects of the invention in analogous manner, independently of any context (process, system, product for computer program, storage unit) concerning them.

A relevant element of the present invention is a storage unit of the units of medicinal products.

By the concept 'medicinal product' or synonymously 'medicine' there shall be understood a substance or a compound of substance destined for the cure or the prevention of human or animal diseases or appropriate to influence physiological functions or to render possible a medical diagnosis.

A medicinal product is habitually produced by a pharmaceutical company and/or introduced into the market as a product ('medicinal product'). Very often the sale of medicinal products is realized by distribution companies, intermediate traders and/or retail shops.

There exist medicinal products solely capable of being sold in some countries to the final clients in specialized shops (for example, chemist's shops). In addition to the medicinal products which may be sold freely (medicinal products not requiring a prescription, OTC (over the counter) products), there exist medicinal products solely saleable in some countries to the final clients by means of the submission of a prescription, being normally prescribed by a doctor (prescription medicinal products). The present invention may be utilized for all of these different products.

Habitually, the medicinal products are distributed in a form packaged together with a so-called directions for use leaflet, wherein there is set out the information for the administration of the medicinal product.

By the concept 'unit of medicinal product' there shall be understood the unit which may be acquired by the final client in a retail shop. In this case it is a matter, for example, of a package wherein a multiplicity of solid portions of the medicinal product (for example, in the form of tablets) is rendered available, exemplifying, in one or various blister packs. It may also be a question of a tube wherein an ointment is stored as a medicinal product. It may be a matter of a glass or plastic bottle wherein the medicinal product is stored, for example in the form of a solution or as a suspension or in the form of capsules or powder. Other examples may be envisaged and obvious to those skilled in the art.

In general, the final client is desirous of receiving a unit of medicinal product. This 'final client' may be the person for whom the medicinal product has been indicated; however it may also be a matter of a person desirous of purchasing a medicinal product for another person or for an animal.

The medicinal products are stored in the form of separate or separable units of medicinal products in a shop of medicinal products (for example, a chemist's shop or a pharmacy) and are rendered available for sale.

They are normally deposited in a storage unit. By the concept "storage unit' there shall be understood, in this manner, a unit which may receive a multiplicity of units of medicinal products and wherein there may be housed the units of medicinal products, having the finality of rendering them available for sale. The examples of a storage unit are a rack, cabinet, case or drawer or similar.

The storage unit according to the invention is habitually prepared by the manufacturer and/or distributor of the medicinal product. This is desirous that the final client acquire the medicinal product thereof and does not select a competitive product, should the medicinal product thereof no longer be available in stock. This manufacturer and/or distributor of the medicinal product is also designated hereinafter as a medicinal product manufacturer (even though not necessarily the manufacturer itself; it may be envisaged that the medicinal product is produced on request by another company). The medicinal product manufacturer is that having the responsibility of introducing the medicinal product into the market. The name thereof is normally affixed upon the medicinal product. Habitually, the medicinal product manufacturer does not have access to the sales systems of the shops selling the products thereof to the final clients. In this manner, it is normal that it also does not possess a general view of the situation of the products and/or of the warehouse and, in this manner, it does not dispose of information regarding the quantity of medicinal products available in a shop.

According to the invention, the medicinal products manufacturer renders available to one or several medicinal products sellers (wherein the sale is made to a final client) a storage unit of the medicinal products thereof by means whereof the medicinal products manufacturer is enabled to remotely consult the quantity of the units of medicinal products situated in the storage unit.

Preferably, the storage unit is configured to be capable of receiving solely the units of medicinal products of the medicinal products manufacturer by whom the storage units will be rendered available. This may be achieved, for example, dimensioning the internal area of the storage unit wherein one or several units of the medicinal products (preferably, in the form of one or several stacks) such that there may fit in solely the units of medicinal products of a defined size. It may be envisaged, exemplifying, that the internal area be dimensioned such that it can receive a stack having a defined quantity of units of medicinal products of a specific medicinal product. Normally, the units of medicinal products present defined dimensions (length, width, height) and form (in the majority of cases, quadrilateral), it being that the internal area of a storage unit may be adapted to these dimensions.

The storage unit according to the invention disposes of resources permitting determining, in an automated manner, the quantity of the units of medicinal products available in the storage unit, together with rendering available the information relating to the quantity to the medicinal product manufacturer. By quantity there shall be understood, habitually, the number of units of medicinal products available.

It may be envisaged, for example, that the units of medicinal products are stored upon shelving as storage unit. In this case, resources would be rendered available permitting determining, in an automated manner, the number of the units of medicinal products situated in the stack.

It may furthermore be envisaged that the units of medicinal products are housed parallelly in a case as storage unit. In this case resources would be rendered available permitting determining, in an automated manner, the number of the units of medicinal products situated parallelly in the case.

By the concept 'automation', for example, as defined by DIN V 19233, there shall be understood 'the equipment of an installation such that it may operate regularly, totally or partially, without the assistance of persons'.

One possibility for calculating, in an automated manner, the quantity of units of medicinal products consists in equipping each unit of medicinal product with an RFID chip wherein there is stored an unequivocal identification. Additionally, the storage unit is equipped with a reading apparatus. The reading apparatus is configured to be capable of reading the RFID chips of the units of medicinal products and consequently determine the quantity thereof, at the defined moments and/or on the registration of the defined events (for example, opening and/or closing of the storage unit).

Independently of whether the units of medicinal products are housed superimposedly or parallelly, in this case reference is made to a stack. A stack has defined dimensions depending on the quantity of the units present in the stack. In a preferred embodiment, the size of the stack is calculated in one dimension (for example, height of the stack or width of the stack), having the objective of determining the quantity of the units of medicinal products available in the stack.

A unit being removed from the stack a lacuna will remain. According to an embodiment of the present invention, the units of medicinal products are disposed (for example, superimposed) such that the lacuna is closed independently (for example, as a consequence of gravity) should units be removed. Upon each removal the size of the stack is reduced in one dimension (exemplifying, height) by virtue of a manner (exemplifying, gravity or elasticity).

In a preferred embodiment, the storage unit disposes of a valve exerting a force upon the stack of the units of medicinal products. Should a unit of medicinal product be removed from the stack, the valve provides for the resultant lacuna to be filled. The force may be generated, for example, by a spring or several springs. A corresponding force may also be exerted through a connecting rod driven by a motor. Other possibilities of the exertion of a force upon the stack are envisagable and known to those skilled in the art.

In a preferred embodiment, the interval of a unit of medicinal product situated at one extremity of the stack is registered as a stationary reference. On the removal of a unit of medicinal product from the stack, the size of the stack is reduced in one dimension (for example, height of the stack or width of the stack) and the interval of the unit of medicinal products situated at one extremity of the stack is altered in relation to the stationary reference. In this manner, the interval represents a moderation for the units of medicinal products available in the stack.

The measurements of the interval may be realized, for example, in a manner being optical (exemplifying, by means of laser motion sensors), acoustic (exemplifying, by means of ultrasonic sensors), inductive, capacitive, mechanical or similar.

In a preferred embodiment, there exists a sensor unit at one extremity of the stack moving jointly with the extremity of the stack when a unit of medicinal product is removed from the stack and the resultant lacuna from that movement is closed, and consequently the size of the stack is reduced in one dimension. The sensor unit registers a physical size being altered when the position of the sensor unit is altered/has been altered.

There exists, furthermore, a sensor unit identifying an alteration in the position and/or an altered position. There may be envisaged, for example, that the sensor unit is situated upon the valve and the position of the sensor unit is altered in relation to a reference marking also existing in the storage unit. The sensor unit registers, in an interaction with the reference marking, a signal depending on the position whereat the sensor unit is precisely situated within the storage unit. Any removal of a unit of medicinal product from the storage unit is associated with an altered position of the sensor unit in relation to the reference marking and is registered by the sensor unit. In this case, it may be envisaged that the sensor unit registers the altered position (the result of the alteration) and/or the process of alteration (the fact that the position is altered during the removal of a unit of medicinal product).

In another embodiment of the present invention, the sensor does not move with the extremity of the stack, it being disposed fixedly in the storage unit in relation to the extremity of the stack, which moves together with the decreasing and increasing height (or width) of the stack. It may be envisaged, exemplifying, that the units of medicinal products exist in the storage unit as stacks, whereupon there is exerted a force upon the stack at one extremity of the stack, pressing the other extremity of the stack against a delimitation in the storage unit, keeping them together whilst, upon a side opposed to the delimitation, there is situated a sensor measuring the interval between the sensor and the extremity of the stack whereupon there is exerted the aforementioned force (displacement sensor).

The alterations of a physical size registered by a sensor may be continuous or discrete. The physical size may be increasing or decreasing when it is a matter of reductive stack size; however there may also be envisaged an alternating increase and/or reduction. A continuous increase or reduction presents the advantage, in comparison with an alternating increase and reduction, that is possible to determine the absolute quantity of the units of medicinal products existing in the stack subsequent to a calibration, whilst an alternating increase and reduction solely indicates the alterations of the size of the stack.

It is also possible to envisage the existence of several sensors/sensor units.

In a preferred embodiment, the sensor unit encompasses a sensor being sensitive to electromagnetic radiation (for example, a photodiode) or a camera chip (for example, CCD or CMOS or similar). Preferably, there exists additionally, adjacent to the sensor unit or separated from the sensor unit, in the storage unit, a source of electromagnetic radiation, preferably in the visible or infrared area, irradiating a reference marking, whilst the sensor registers the radiation reverberated (reflected) by the reference marking (or by at least one part thereof).

The reference marking is generated, preferably, through the alteration of a physical size when, as a consequence of the reduction of the size of the stack, another part of the reference marking is irradiated. It may be envisaged, for example, that the quantity of reflected radiation increases or is reduced when the size of the stack diminishes. It is also possible to envisage that the spectral distribution of the reflected radiation is altered when the size of the stack diminishes. It may also be envisaged that the electromagnetic radiation goes in another sense when the size of the stack diminishes. Consequently, the sensor is executed such that the altered sense is identified, for example, such that the reflected radiation may come from another direction.

A preferred embodiment is when there exists a sensor unit affixed upon a valve. The valve provides, through a force acting upon an extremity of the stack, that the units of medicinal products be kept together in the stack. A unit of product being removed, the resultant lacuna is automatically closed by the valve. The sensor unit always moves together with the valve. When the valve assumes a new position as a consequence of the removal of the unit of product, the sensor unit affixed upon the valve also assumes a new position. The storage unit comprises, furthermore, a reference marking. This marking is stationary, that is to say the position of the reference marking is not influenced by an alteration in the size of the stack and by an altered position of the valve and of the sensor unit. The size of the stack being altered, the position of the sensor unit is also altered in relation to the reference marking. The sensor unit disposes of a source of electromagnetic radiation having one or several wavelengths in the visible light area (380 nm to 78 nm) and/or in the infrared radiation area (780 nm to 1 mm). The source may be, for example, a light emitting diode. It is also possible to envisage that several sources exist. The source sends the electromagnetic radiation in the direction of the reference marking. One part of the resultant radiation is reverberated (reflected) by the reference marking. A part of the reflected radiation reaches a sensor sensitive to the electromagnetic radiation. The reference marking is consequently generated such that the fraction of radiation reflected is altered along the length of the reference marking. This may be achieved, for example by means of an increasing or decreasing degree of darkening. The darker the respective area of the reference marking whereupon the electromagnetic radiation from the source is manifested, the smaller will be the fraction reflected and the greater will be the fraction absorbed. Should the position of the sensor unit be altered in relation to the reference marking, the quantity of radiation manifested upon the sensor will be altered. Subsequent to the calibration, it is possible to deduce the quantity of units of medicinal products through the quantity of radiation detected.

In another preferred configuration, a displacement sensor affixed in the opposite sense to one extremity of the stack of the units of medicinal products is utilized. The displacement sensor measures the interval between the displacement sensor and the extremity of the stack. Should a unit of medicinal product be removed from the stack, the stack is pressed in a direction remote from the displacement sensor. In this manner the interval is increased, being registered by the displacement sensor.

In addition to a sensor (or several sensors) registering a physical size associated with the quantity of units of medicinal products (for example, correlated) there exists, preferably, a microcontroller or similar, by means whereof the physical size (possibly subsequent to the calibration) may be translated into a quantity of units of medicinal products. The microcontroller may be an integral component of the sensor or of the sensor unit or may be a separate element situated adjacently to or in the proximity of the storage unit.

Other resources to determine the quantity of portions of medicinal products in a storage unit are revealed, for example in the publication US2005/168345A1, the content whereof is adopted in this description by reference.

It may be envisaged that the quantity of the units of medicinal products available in a storage unit is continuously registered. It is also possible to envisage that this quantity is registered at defined moments, for example periodically (exemplifying, every minute). It is also possible to envisage that a registration will be effected when the quantity is or has been altered, for example resulting form the removal of a unit of medicinal product from the storage unit.

In a preferred embodiment, the registration is effected when the units of medicinal products are removed and/or added to the storage unit. Preferably, the moment is registered whereat there is removed and/or added one or several units of medicinal products, registering the quantity of the units of medicinal products removed and/or added. It may furthermore be envisaged that the person removing and/or adding the units of medicinal products will be registered. It may be envisaged, for example, that the storage unit is closed and a person shall have access, for example, solely through the aid of an identity card prior to it being possible to open the storage unit.

The storage unit according to the invention is connected, in a communicative manner, to a first computerized system. Through this communicative connection, the storage unit is capable of transmitting the quantity of each unit of medicinal product available to the first computerized system.

The first computerized system is normally a stationary computerized system whereto are connected several storage units. The first computerized system is not situated habitually in a shop wherein the medicinal products are sold to the final clients. Normally, the manufacturer of medicinal products has control over the first computerized system.

The connection between the first computerized system and the storage units may be realized, for example, by a network. The connection may be realized by a mobile and/or stationary network. It may be realized by means of cables and/or radio connection.

It may be envisaged that diverse storage units transmit the respective quantities of units of medicinal products to a central hub by means whereof the information is sent to the first computerized system. The communication with the hub may be realized, exemplifying, through a method of short range data transmission (for example, by radio through a model such as Bluetooth or Zigbee or similar), and sending may be realized through a long-range data transmission system (for example, fiber optic network or cable network).

The first computerized system is connected to a data base. The data base may be a component of the first computerized system. It may however also be a data base being a component of another computerized system whereto the first computerized system may be connected by means of a network. It is possible to envisage, for example, that the data is situated in the cloud. Cloud computing describes the rendering available of IT infrastructure, such as for example memory space, processing capacity or application software by means of the Internet.

There are stored, in the data base, the premises wherein the storage units are situated. There is also registered, for each storage unit, the quantity of units of medicinal products available in each storage unit. It may be envisaged that the data base is periodically updated every second, every minute, every 10 minutes, every hour or at other moments. Updating signifies that the data stored in the data base is adjusted to the reality. It is also possible to envisage that the updating of the data base be brought about by an event. On the occurrence of a corresponding event, the alterations to the reality are admitted totally or partially into the data base. An event of this nature may be, exemplifying, an altered stock of units of medicinal products in storage unit. An event of this nature may be, for example, a consultation by a user: if a user inputs, exemplifying, a premises and a medicinal product in the search field of a search mask in a second computerized system, the data base will be updated in relation to the premises situated in a defined vicinity of those premises in order that the user may receive information regarding the current stock in real time.

The consultation regarding the stock of units of medicinal products in a defined vicinity of the premises is realized by a second computerized system operated by a user. The second computerized system is a stationary computerized system (for example, a desk top computer or a terminal) or a mobile computerized system (for example, a tablet computer, a smart phone or a lap top or similar).

The second computerized system is configured such that the person may receive information regarding the stock of units of medicinal products in a defined vicinity of a premises through the first computerized system and/or from the data base. Should the data be located in the cloud, it is possible that the second computerized system may directly access the data base. Should the data base be an integral component of the first computerized system, the access is realized preferably by the first computerized system.

The consultation regarding the stock is habitually realized by means of a graphical user interface. This interface is generated, exemplifying, by a computer program (for example by a so called app (application) or by a web browser or similar).

In a consultation of this nature there is calculated, in a first stage, the medicinal product whereof the user is desirous. The user will be enabled to input the name of the medicinal product, for example in a search field by means of a keyboard or by voice command by means of a microphone. It may be envisaged, also, that the user will select the medicinal product from a listing (for example, by means of a mouse or by means of a finger upon a touch pad). It is also possible to envisage that the user will have an optical code or similar (for example, upon packaging of a product purchased or upon a receipt or a leaflet). It may be envisaged that the user will input the optical code by means of a camera, being preferably a component of the second computerized system, and a computer program will interpret the optical code in a second computerized system, the latter identifying the medicinal product associated with the optical code.

It may be envisaged that there will be indicated to the user diverse medicinal products referring to a selected medicinal product (for example, diverse sizes of packaging and/or concentrations in relation to an active principle and/or diverse forms of application and/or diverse formulations and/or diverse combined preparations) whereof the person may select one or several.

It is also possible to envisage that the user presents symptoms and inputs those symptoms or a disease by means of the registration resources in the second computerized system, there being subsequently indicated thereto medicinal products for the treatment of the symptoms or of the disease.

In a second stage (also being realizable initially prior to the first stage) a premises is calculated. In this case, preferably, it is a matter of a premises located in the accessible proximities of the user and in the vicinity whereof the user would like to acquire the medicinal product. In an embodiment, this premises is the premises wherein the user is precisely situated on utilizing the second computerized system. This premises may be calculated, for example, automatically, wherein there will be calculated, for example, the GPS coordinates of the second computerized system. Should the second computerized system be connected to a mobile network, the approximate location of the user will also be capable of being determined by means of the calculation of the radio cell wherein the user is precisely situated.

In a preferred embodiment, the user inputs a premises in a graphical user interface mask. The inputting may be realized, for example, by means of the keyboard or by means of voice command through the microphone. The premises may be specified, exemplifying, by means of an address (state, city, street, house number) or by means of the data of the line of length and of width. It is also possible to envisage that there is displayed to the user a digital card whereupon the user may indicate the premises, for example, by means of a mouse cursor or mouse click. Selecting a premises by means of the selection menu may furthermore be envisaged.

The premises having been calculated, it is determined whether the storage units, especially the storage units of the medicinal product desired, should this be already known, are situated in a defined vicinity. The defined vicinity may be, for example, the vicinity within a perimeter surrounding the premises. It may be envisaged that a value for the radius of the perimeter (for example, 500 meters, 1 kilometer, 10 kilometers, 25 kilometers or other values) has been preset. Preferably, the vicinity may be set by the user, for example, inputting or selecting a radius of a perimeter or, for example, marking a vicinity by means of a mouse cursor or by means of the finger upon a touch pad screen.

In another stage, there is calculated, through consultation of the data base, whether storage units exist in the defined vicinity of the premises calculated/selected/input, whether they dispose of the medicinal product desired and, in the affirmative, how many units of medicinal product are available in the respective storage units.

The locations of these storage units containing units of the medicinal product desired are displayed to the user together with the units of medicinal product contained in the storage units. The display may be realized in one or more listings, for example. Preferably, there is displayed to the user a digital map whereupon the premises of the storage units are located. The display may be supplemented by signposting colors, for example those premises wherein a quantity of units of medicinal product is present exceeding a definable or defined limit are, for example, in green, whilst the premises wherein a quantity of units of medicinal products available is below a definable or defined threshold, for example, intense yellow, whilst the premises wherein there are no units of medicinal products present are marked in red, for example.

In a manner of preferred embodiment, there is displayed to the user for all the locations presented or for selected premises the quantities will be of units of medicinal product subsequent to a defined or definable period in accordance with exists prediction. For example, the prediction may be acquired from a statistical analysis of changes in the stock in the past and modeling of the results. In this manner, the user may verify not solely whether the quantity is currently available but also whether there are sufficient units of medicinal products available subsequent to the user having arrived at the appropriate premises.

In a manner of preferred embodiment, there is determined the location of the user, the location of the proximate storage unit wherein the unit of product is, at least, an available medicinal product, how long the user would require on foot, in a car, upon a motor bicycle, a bicycle, by public transport and/or other means and/or a combination of different types of transport, to reach the storage unit, the number of units of medicinal products that are sold as expected at that moment by virtue of numbers of past sales and the number of units of medicinal products at the moment of the users reaching the storage unit in the storage unit which will still be present. The quantity of units of medicinal products it is expected will be present may be displayed to the user.

It is also conceivable that the system be configured such that the user may make a reservation such that the quantity desired be covered and be available when the user reaches the corresponding storage.

A display for a user is generally realised by means of suitable characters, texts, tags, images, graphics, digital maps and similar upon a screen. It is also conceivable that a linguistic (acoustic) "display" is realised, for example for a user, the addresses of the localisations wherein the desired pharmaceutical products are to be the test, together with the respective quantities in stock.

It is also conceivable to incorporate the consultation for units of medicinal products in stock into a dialogue with a chat bot. For example, it is conceivable that a user be seeking a medicinal product to alleviate the symptoms thereof. The user asks a chat bot on a site for possible medicinal products which may be of assistance thereto. The chat bot proposes one or more products thereto. The user selects a product. The user is desirous of knowing where they may purchase the product. The localisation of the user is determined and the chat bot displays the shops proximate to the premises, together with the quantities in stock.

The system may also be used by the medicinal product manufacturer, for example, displaying the distribution of the quantities on line upon a digital map for a city, region or country. This information may assist it in optimising its sales and logistics.

In a manner of particularly preferred embodiment, the system is configured to notify the manufacturer of the medicinal product when the quantity of units of medicinal product in a shop and/or region are below a defined threshold. It may then take measures to prevent one or more shops from storing units of medicinal products.

The invention is explained in greater detail below through reference to the figures, without it being the intention to limit the invention to the manners of embodiment illustrated or to the characteristics illustrated in the figures. The figures shall be understood as merely preferred manners of embodiment.

There are shown:

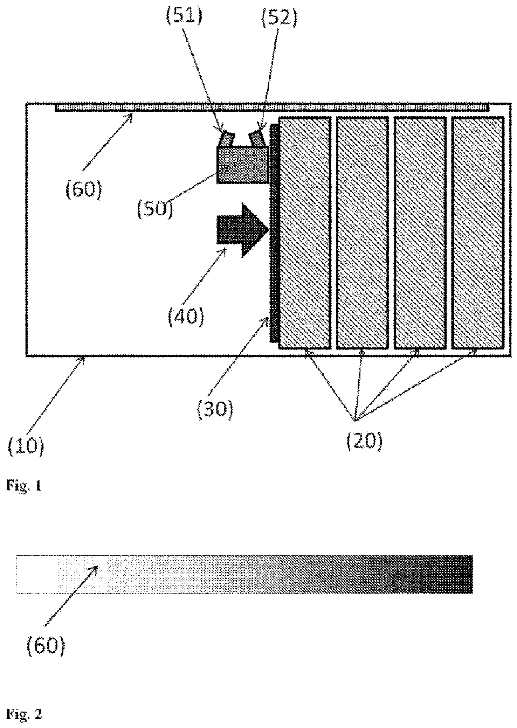

Figure 1 shows schematically a manner of preferred embodiment of a storage unit according to the invention.
   The storage unit (10) includes four units of medicinal products (20) disposed in a stack. One unit of medicinal product (on the right in Figure 1) forms one extremity of the stack, another unit of medicinal product (to the left in Figure 1) forms the other extremity of the stack. At one extremity of the stack there is a pusher (30). The traveler (30) exerts a force (represented by the arrow (40)) upon the stack and, in this manner, it is kept together. If a unit of medicinal product (20) is removed from the stack, the resultant lacuna is closed again by the force (40). The pusher moves in the direction wherein the force (40) acts. The pusher is connected to a sensor unit (50) which always moves with the pusher. The sensor unit (50) has a source of electromagnetic radiation (51) and a sensor (51) sensitive to the electromagnetic radiation. Upon the internal wall of the storage unit (10) there is affixed a reference mark (60). When a unit of medicinal product (20) is removed from the stack, the sensor unit (50) moves together with the traveler (30) in the figure to the right. As a result, the source (51) irradiates another part of the reference mark (60). The reference mark presents a different nature as a function of the length thereof (see, for example, Figure 2). The different nature leads to a change in the reflection of the electromagnetic radiation, this being registered by the sensor (52).
Figure 2 shows schematically a preferred manner of embodiment of the reference marking of Figure 1.
   Whilst the reference mark (60) is shown in Figure 1 in a plan view, the reference mark (60) is shown in Figure 2 in a side view, facing the source (51) and the sensor (52) of Figure 1, such that the electromagnetic radiation emitted by the source reaches the side shown in Figure 2, is reflected from that side and, subsequently, reaches the sensor. The reference mark (60) presents an increasing degree of darkening to the right. The electromagnetic radiation reaching a region presenting a lower degree of darkening is reflected to a greater extent than the electromagnetic radiation reaching a region presenting a higher degree of darkening. Consequently, the reflectance detected by the sensor (52) decreases from the left to the right. The less units of medicinal products (20) present in the stack the less electromagnetic radiation is reflected from the reference mark (60) and registered by the sensor (52). Subsequent to the calibration of the sensor unit (50) absolute statements may be made regarding the quantity of units of medicinal products (20) present in the storage unit (10).

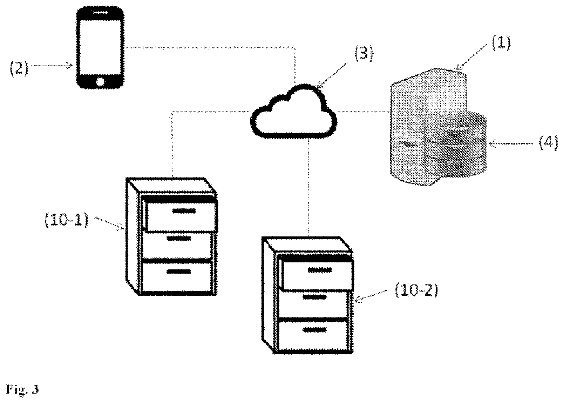

Figure 3 shows schematically a manner of preferred embodiment of the system of the invention, comprising a plurality of storage units (10-1, 10-2) for units of medicinal product, a first computer system (1), a data base (4) and, at least, a second computer system (2). The storage units (10-1, 10-2) are located in different premises. They possess means to register the quantity of units of medicinal products present in the respective storage units. They are in a communicative connection with the first computer system (1) by means of a network (represented by dashed lines) and the cloud (3). They are configured to communicate the respective quantities of the units of pharmaceutical products present in the storage units to the first computer system (1). The first computer system (1) is configured to receive and store in the data base (4) the quantities of units of medicinal products present in the storage units (10-1, 10-2). In the data base (4) there are stored the premises wherein the storage units (10-1, 10-2) are located and the quantities of units of medicinal products present in the storage units (10-1, 10-2). In the present example, the data base (4) is directly connected to the first computer system (1). Nevertheless, it is also conceivable that the data base (4) be located in another premises and connected to the first computer system (1) by means of a network (for example (3)). The second computer system (2) is executed in the present example in the form of a smart phone. It is connected to the first computer system and/or to the data base by means of the network (3). It is configured such that a user, subsequent to inserting and/or selecting a premises, or subsequent to detecting the premises wherein the user is located at the moment of inserting information regarding a medicinal product or subsequent to selecting a medicinal product, the defined premises it retains the storage units in the vicinity of the premises and the number of each one thereof contained in the units of pharmaceutical products of the storage units. This information is supplied by the data base (4).

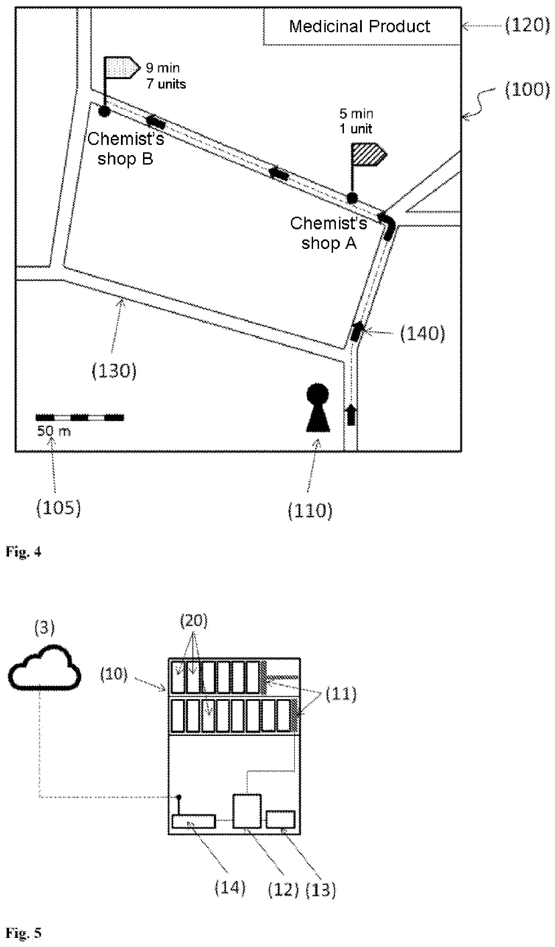

Figure 4 illustrates schematically an example of a display presented upon a screen of the second computer system for a user of the second computer system.
   A general map (100) is shown upon the screen. A user icon (110) indicates the location of the user. The roads are marked in the form of line trains (130). A legend (105) indicates the scale (50 m). In the upper right hand corner is the name (120) of the medicinal product desired by the user and the user wishes to know whether it is available proximate to the location thereof.
   The map (100) shows two shops (Chemist's shop A, Chemist's shop B) by means of flags showing the corresponding pharmaceutical product. Beside or above the flag it indicates how many units of medicinal products are available in each shop (1 unit, 7 units) and how long the user will take from the location thereof to reach the respective shop (5 minutes, 9 minutes). It is also conceivable to specify how many units of medicinal products in the respective shop should be available at the moment whereat the user arrives at the respective shop. Dashed lines and arrows (140) indicate the way whereby the user may reach the shops as quickly as possible. In the present example, all the information is in English. Nevertheless, it is conceivable that information may be written in another language or in several languages.
Figure 5 shows schematically an example of a manner of preferred embodiment of the storage unit according to the invention.
   The storage unit (10) contains two rows of a plurality of units of medicinal products (20). The storage unit (10) possesses means (11, 12) to detect the quantity of units of medicinal products (20) present in the storage units. A sensor unit (11) detects the quantity of units of medicinal products (20) disposed in a row. The sensor unit (11) is connected to a control unit (12) detecting and evaluating the signals from the sensor unit. The results are transmitted by means of a wireless transmission unit (14) to a network (3). The electric or electronic components are supplied with electrical energy by means of a source of supply (13). It is conceivable that the storage unit (10) be connected to the energy network of the business wherein it is located. It is also conceivable that the energy supply unit (13) be a mobile energy supply unit, for example a battery or an accumulator. A mobile energy supply unit presents the advantage of independence from external sources of energy; in general, the storage unit is supplied by the manufacturer and/or distributor of the corresponding pharmaceutical products and not by the owner/operator of the shop.

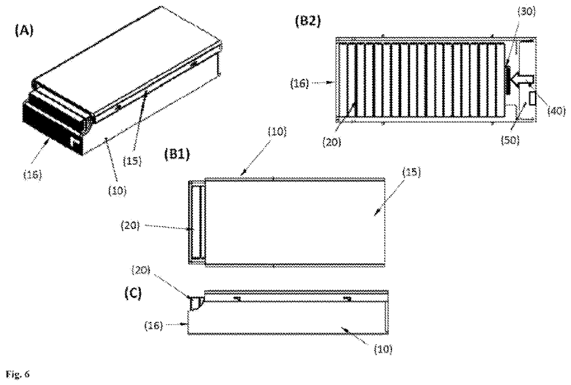

Figure 6 shows schematically another example of a storage unit according to the invention in a perspective view (A), in a plan view (B1) with lid (15), in a plan view (B2) without lid, and in a side view (C).
   The storage unit (10) is dimensioned to receive a stack of 20 units of medicinal product (20) (in the illustration B2, the stack 19 comprises units of medicinal product such that the stack may still accommodate a further unit of medicinal product). By virtue of the size and shape of the interior of the storage unit (10), adapted to the size of a stack of units of medicinal products of a specific medicinal product, the storage unit (10) may be used solely for that medicinal product. The units of medicinal products are kept together in the stack by a pushing device (30); the traveler (30) pushes the stack against a limit (16). The direction of the force acting upon the stack is indicated by the arrow (40). If the storage unit (10) with the cover (15) is closed, only the unit of medicinal product may be removed from the storage unit (10) bearing against the limit (16). When the unit of medicinal product is removed a gap is created, being closed by the slide (30) moving in the direction of the constraint (16) by virtue of the force (40). This increases the distance between the extremity of the stack in contact with the traveler (30) and a distance sensor (50) measuring this distance.
   A storage unit according to the invention may be conceived such as to be combined with other storage units to form a stack. For example, several of the storage units shown in figure 6 may be stacked one upon another.

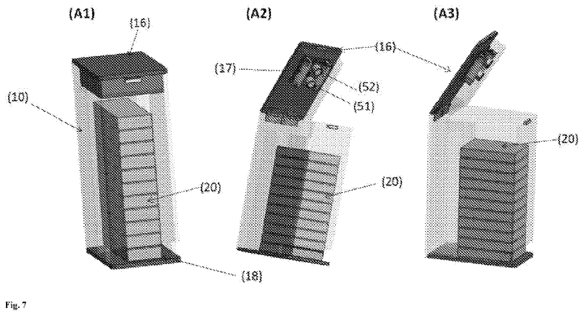

Figure 7 shows schematically another example of a storage unit according to the invention in three perspective views (A1, A2, A3). The walls of the storage unit (10) are shown in a transparent manner for better comprehension. In the illustration (A1), the lid (16) is closed, in the illustrations (A2, A3) it is open. In the storage unit (10) there exists a stack of 11 units of medicinal products (20). The stack is kept together by the walls and by gravity. A unit of medicinal product is removed by means of the aperture (18) in the bottom of the storage unit. On removing a unit of medicinal product, the distance between the upper extremity of the stack and the lid (16) increases. Within the interior of the lid a sensor unit is mounted. The sensor unit is composed by a distance sensor. There may be observed a source (51) for electromagnetic radiation and a receiver (52). In addition thereto, a source of supply (17) in the form of two batteries may be observed. The sensor unit furthermore includes a control unit and a transmission unit (not shown). The sensor unit is configured to determine and to communicate to a computer system the number of units of medicinal products within the storage unit on the basis of the distance between the lid and the top of the stack.

## Claims

1. A SYSTEM, **characterized in that** it comprises:
- a plurality of storage units of units of medicinal products,
the storage units possessing means to detect the quantity of units of medicinal products present in the respective storage unit,
wherein the storage units are in communication with a first computer system and are configured to communicate the respective quantities of the units of medicinal product present in the storage units to the first computer system,
- the first computer system,
being communicatively connected to the storage units and configured to receive and store in a data base the quantities of units of medicinal products present in the storage units;
- the data base,
wherein are stored the premises wherein the storage units are located and the respective quantities of units of medicinal products in existence received for the respective storage units;
- at least a second computer system,
communicating with the first computer system and/or the data base by means of a network in a communicative connection, and configured such that a user enters and/or selects a premises or subsequent to the detection of the location whereat the user is actually located, and following the inputting of information regarding a medicinal product or the selection of a medicinal product, the locations of the storage units located in a defined environment for the location and the number of respective units of medicinal products present in the storage units.

2. The SYSTEM according to claim 1, **characterized in that** at least one part of the storage units being storage units according to any one of claims 13 to 15.

3. The SYSTEM according to claim 1, **characterized in that** it is configured to provide the user with a forecast of which quantities of units of medicinal products will still be available subsequent to the expiry of a defined period in the respective locations.

4. The SYSTEM according to claim 1, **characterized in that** it is configured to present to a medicinal product manufacturer the units of medicinal products in a region upon a digital map and to receive a notification when the quantities in one or more locations are below a threshold. to decline.

5. A METHOD, **characterized in that** it comprises the stages of:
- supply of storage units containing units of medicinal products in different premises,
- automated recognition of quantities of units of medicinal products lying in the respective storage units,
- transmission of the quantities to a first computer system,
- recording of the quantities and premises in a data base,
- provision of access to the first computer system and/or to the data base for a user by means of a second computer system, wherein the access is configured such that subsequent to the inputting and/or selection and/or detection of a premises, the user is displayed in those premises having storage units located in a defined environment for the premises and, in addition to the user, the quantities of the units of medicinal products existing are shown.

6. The METHOD according to claim 5, **characterized in that** at least one part of the storage units are storage units according to any of the claims 13 to 15.

7. The METHOD, **characterized in that** it comprises the stages of:
- inputting and/or selection of a medicinal product by a user,
- inputting and/or selection of a premises by a user and/or detecting the premises whereat the user is located,
- display of premises in a defined environment for the premises, wherein the premises contain storage units containing the medicinal product and displaying the quantity of units of medicinal products in the respective storage units for the user.

8. The METHOD according to claim 7, **characterized in that** there is presented to the user a forecast of what quantities of units of medicinal products will still be available subsequent to a defined period in the respective locations.

9. The METHOD according to any one of the claims 7 to 8, **characterized in** at least a part of the storage units are storage units according to one of the claims 13 to 15.

10. A COMPUTER PROGRAM PRODUCT, **characterized in that** it comprises a data registering device wherein a computer program is stored loadable into the main memory of a computer system and making the computer system execute the following stages of:
- capturing a premises,
- gathering information regarding a medicinal product,
- determining premises in a defined environment for the premises wherein the storage units of medicinal products are located,
- determining the quantities of units of medicinal products which are, respectively, in the storage units,
- displaying the premises and the quantities of units of medicinal products available in each site for a user.

11. The COMPUTER PROGRAM PRODUCT according to claim 10, **characterized in that** the computer program product is configured to execute one or more stages of the method according to any one of the claims 7 to 9.

12. A STORAGE UNIT FOR UNITS OF MEDICINAL PRODUCTS, **characterized in that** it presents storage unit means to detect the quantity of units of medicinal products present in the storage unit,
wherein the storage unit is in communication with a first computer system and configured to communicate the quantity of the unit of medicinal product present in the storage unit to the first computer system.

13. The STORAGE UNIT according to claim 12, **characterized in that** the quantity of medicinal product existing in the storage unit is determined on the basis of the requirements of space thereof.

14. The STORAGE UNIT according to any one of the claims 12 to 13, **characterized in that** it comprises:
- a space to receive a stack of units of medicinal products,
- a traveler applying force upon the stack and, in this manner, keeping the units of the pharmaceutical product together in the stack, the position of alteration of the traveler in relation to a reference in the storage unit when a unit of medicinal product is removed from the stack.

15. The STORAGE UNIT according to any one of the claims 12 to 14, **characterized in that** it comprises a sensor unit, wherein the sensor unit comprises a source of electromagnetic radiation and a sensor sensitive to the electromagnetic radiation, **characterized in that** the source of electromagnetic radiation irradiates a reference mark and the sensor detects at least a part of the reflection of the radiation from the reference mark, wherein the reference mark presents a variable reflectivity along the length thereof towards the radiation.
